# FASCICULE DE BREVET EUROPEEN

(11) **EP 1 084 126 B1**
(45) Date de publication et mention de la délivrance du brevet: **17.11.2004**
(21) Numéro de dépôt: 99922250.8
(22) Date de dépôt: 02.06.1999
(51) Int. Cl.: C07D 519/04

(54) **NOUVEAUX DERIVES DE VINCA-ALCALOIDES ET PROCEDES DE PREPARATION**
NEUE VINCA-ALKALOIDDERIVATE UND VERFAHREN ZUR HERSTELLUNG
NOVEL VINCA-ALKALOID DERIVATIVES AND PREPARATION METHOD

(30) Priorité: 02.06.1998 FR 9806895
(43) Date de publication de la demande: 21.03.2001
(73) Titulaire: Roowin, S.A., 75013 Paris (FR)
(72) Inventeur: ROOL, Patrice, F-91800 Brunoy (FR)
(74) Mandataire: Nargolwalla, Cyra
(86) Numéro de dépôt international: PCT/FR1999/001289
(87) Numéro de publication internationale: WO 1999/062912

(56) Documents cités:
- WO-A-89/12056
- DE-A- 3 801 450
- DE-A- 3 826 412
- US-A- 5 037 977
- RICHARD J. SUNDBERG ET AL.: "Mechanistic aspects of the formation of anhydrovinblastine by Potier-Polonovski oxidative coupling of catharanthine and vindoline. Spectroscopic observation and chemical reactions of intermediates" TETRAHEDRON., vol. 48, no. 2, - 10 janvier 1992 (1992-01-10) pages 277-296, XP002083507 OXFORD GB
- RICHARD J. SUNDBERG ET AL.: "Oxidative fragmentation of catharanthine by dichlorodicyanoquinone" JOURNAL OF ORGANIC CHEMISTRY., vol. 56, no. 5, - 1 mars 1991 (1991-03-01) pages 1689-1692, XP002083508 EASTON US
- RICHARD J. SUNDBERG ET AL.: "Photoactivated C16-C21 fragmentation of catharanthine" TETRAHEDRON LETTERS., vol. 32, no. 26, - 24 juin 1992 (1992-06-24) pages 3035-3038, XP002083509 OXFORD GB

## Description

L'invention concerne un procédé de préparation de vinca-alcaloïdes de formule générale (I) 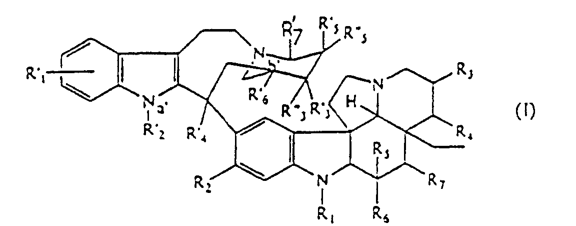 dans laquelle :
- R'₁ représente un atome d'hydrogène ou un radical alcoxy, acyle, formyle ou halogénoacyle,
- R'₂ représente un atome d'hydrogène ou un radical alcoyle,
- R'₃ et R"₃ sont identiques ou différents l'un de l'autre et représentent chacun indépendamment un atome d'hydrogène ou un radical hydroxyle, alcanoyloxyle, ou bien R'₃ et R"₃ forment ensemble un groupement carbonyle, ou bien R'₃ et R'₅ forment ensemble un pont époxy ou une double liaison,
- R'₄ représente un atome d'hydrogène ou un radical alcoyloxycarbonyle, hydroxyméthyle ou alcanoyloxyméthyle, de préférence alcoyloxy carbonyle,
- R'₅ et R"₅ sont identiques ou différents l'un de l'autre et représentent chacun indépendamment un atome d'hydrogène ou un radical hydroxyle, alcanoyloxyle, éthyle ou hydroxy-2-éthyle,
- R'₆ représente un atome d'hydrogène ou un radical éthyle, hydroxy-2-éthyle ou acétyle,
- R'₇ représente un atome d'hydrogène ou un radical cyanure,
- R₁ représente un atome d'hydrogène ou un radical alcoyle, formyle ou acyle, de préférence hydrogène ou alcoyle,
- R₂ représente un atome d'hydrogène ou un radical alcoxy,
- R₃ représente un atome d'hydrogène ou un radical hydroxyle, alcanoyloxyle, ou bien R₃ et R₄ forment ensemble un pont époxy ou une double liaison,
- R₄ représente un atome d'hydrogène ou un radical hydroxyle, alcanoyloxyle, ou bien R₄ et R₅ forment ensemble un pont époxy,
- R₆ représente un radical alcoyloxycarbonyle, hydrazido, acétamido, hydroxyméthyle ou alcanoyloxyméthyle,
- R₅ et R₇ représentent un atome d'hydrogène ou un radical hydroxyle ou alcanoyloxyle,
ainsi que leurs sels d'addition avec les acides et leurs sels d'ammonium quaternaire.

Certains dérivés de la formule (I) sont connus comme étant des intermédiaires dans la préparation des médicaments anti-tumoraux tels que la vinblastine, la vincristine et la vinorelbine.

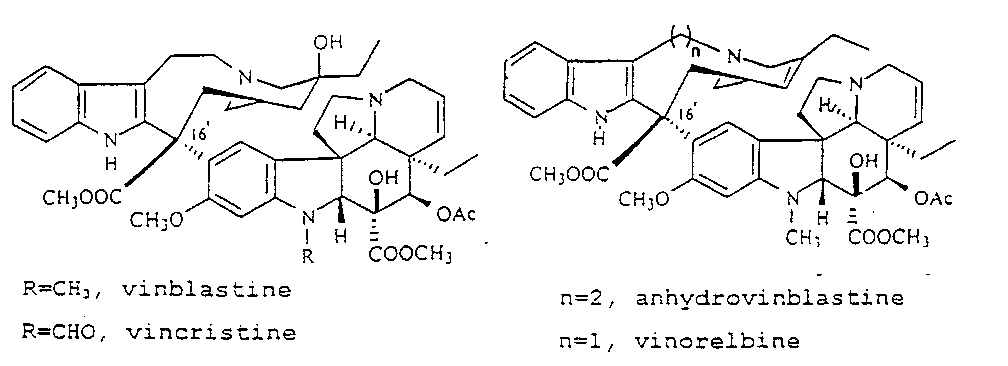

Les propriété anti-tumorales remarquables de ces molécules naturelles complexes, extraites de la Pervenche de Madagascar, Catharanthus roseus, sont connues et elles sont déjà utilisées en traitement anti-cancéreux. La vinblastine et la vincristine sont des "poisons de fuseau" qui s'opposent à la formation du fuseau mitotique lors de la division cellulaire, empêchant ainsi la prolifération cellulaire.

La vincristine et la vinblastine sont des agents actifs dans le traitement des leucémies, des lymphosarcomes et des tumeurs solides. La vinblastine est également utilisée dans le traitement de la maladie de Hodgkin.

La vinorelbine est actuellement utilisée dans le. traitement de la forme la plus répandue du cancer des poumons à savoir le cancer du poumon à cellules non petites. Elle est également utilisée dans le traitement des cancers métastasiques du sein.

Les méthodes actuellement employées pour la préparation de la vinblastine et de la vincristine impliquent l'extraction de ces molécules à partir des plantes. Celles-ci peuvent être broyées et séchées avant que ces substances puissent en être extraites. Le procédé d'extraction est long et coûteux étant donné que l'extrait obtenu est très complexe, contenant au moins 200 alcaloïdes différents. Les rendements sont également très faibles, on obtient 5 à 10 g de vinblastine par tonne de plantes séchées, et 0,5 à 1 g de vincristine par tonne de plantes séchées.

De nombreux groupes de recherche ont donc essayé de réaliser la synthèse de ces molécules en utilisant des procédés plus efficaces qui permettraient de meilleurs rendements, et qui mettraient en oeuvre des dérivés ayant des propriétés anti-tumorales intéressantes mais dotées d'une toxicité moindre.

Le brevet FI 882 755 déposé par la société HUATAN-MAKI Oy concerne la formation de la vinblastine et de la vincristine par irradiation de la catharanthine et de la vindoline, avec des rayons UV dans une solution aqueuse acide, sous une atmosphère d'oxygène ou de gaz inerte. Les rendements obtenus dans ces réactions sont extrêmement faibles.

Par ailleurs, d'autres procédés sont connus qui mettent en oeuvre l'anhydrovinblastine qui est un intermédiaire de synthèse pour la vinblastine, la vincristine, ainsi que la vinorelbine.

L'anhydrovinblastine est donc un intermédiaire chimique clé qui permet d'accéder à l'ensemble des alcaloïdes de type vinblastine. Cet intermédiaire est synthétisé par un couplage de la catharanthine et de la vindoline.

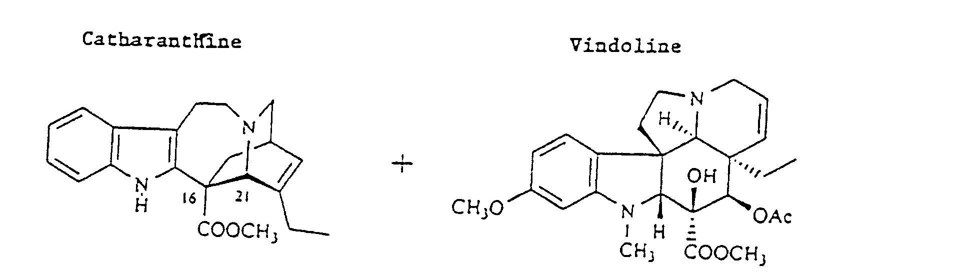

Ces deux derniers alcaloïdes sont également extraits de le Pervenche de Madagascar, mais contrairement à la vincristine et à la vinblastine ils représentent les constituants principaux de l'extrait obtenu. En effet, on obtient 400 g de catharanthine par tonne de plantes séchées et 800 g de vindoline par tonne de plantes séchées

La préparation de l'anhydrovinblastine par le couplage de la catharanthine et de la vindoline constitue donc une voie privilégiée de synthèse de ce produit intermédiaire.

Il existe plusieurs procédés pour la préparation de l'anhydrovinblastine à partir de la catharanthine et de la vindoline.

Le brevet FR 2 296 418 déposé par l'ANVAR les demandes DE 3801450 et DE 3826412 et l'article The Mechanistic aspects of the formation of anhydrovinblastine by Potier - Polonovski oxidative coupling of catharanthine and vindoline. Spectroscopic observation and chemical reactions of intermediate" Tetrahedron. Vol 48, no 2. 10 January 1992 pages 277-296. Richard J. Sundberg et al. décrivent un procédé au cours duquel le N-oxyde de catharanthine est couplé à la vindoline en présence de l'anhydride trifluoroacétique.

Lorsque ce procédé est réalisé à température ambiante, seul l'épimère 16'-R non actif de l'anhydrovinblastine est obtenu. L'épimère naturel actif 16'-S est obtenu majoritairement lorsque cette réaction est effectuée à une température inférieure à moins 50°C sous gaz inerte. Cependant, même à basse température, il subsiste 10% de l'épimère 16'-R de l'anhydrovinblastine.

Ce procédé présente des inconvénients importants. Les conditions opératoires sont extrêmement contraignantes à cause de l'utilisation de solvants anhydres, de la basse température, et de l'atmosphère de gaz inerte. Le produit obtenu doit être soumis à une opération de purification de par la présence de 10% de l'épimère 16'-R de l'anhydrovinblastine. Le rendement en anhydrovinblastine isolé est faible, de l'ordre de 35%.

Un deuxième procédé proposé par VUKOVIC et al. dans la Revue "Tetrahedron" (1998, volume 44, pages 325-331) propose un couplage de catharanthine et de vindoline initié par des ions ferriques. Il s'agit également d'une oxydation de la catharanthine. Le rendement en anhydrovinblastine est de l'ordre de 69% lorsque la réaction est conduite sous une atmosphère de gaz inerte. Ce procédé comporte néanmoins l'inconvénient majeur de conduire à de nombreux produits secondaires. Il s'agit d'impuretés résultant de la suroxydation des alcaloïdes dimériques formés quelles que soient les conditions opératoires choisies. Ceci rend donc l'étape de purification difficile et délicate.

Un procédé amélioré a été proposé dans le brevet US 5 037 977 qui augmente le rendement en anhydrovinblastine jusqu'à 89%. Cependant, cette amélioration n'est décrite que sur des quantités de réactifs très faibles et son extension à une échelle industrielle semble difficile. En tout état de cause, ces procédés à base d'ions ferriques conduisent dans tous les cas à de nombreux produits secondaires du fait que ces ions sont responsables de réactions parasites.

Un troisième procédé décrit par GUNIC et al dans "Journal or the Chemical Society Chemical Communications" (1993), volume 19, pages 1496-1497, et Tabakovic et al. dans "Journal of Organic Chemistry" (1997), volume 62, pages 947-953, décrit une réaction de couplage de la catharanthine et de la vindoline par une oxydation anodique de la catharanthine. Cependant, ce procédé souffre également d'inconvénients qui sont d'une part de nécessiter une atmosphère inerte, et, qui sont d'autre part, liés à la nature même du procédé électrochimique impliquant l'usure des électrodes, le contrôle difficile de la reproductibilité, le coût des électrolytes. Et, tout comme dans les approches précédentes, l'anhydrovinblastine est contaminée par environ 10% de l'épimère 16'-R anhydrovinblastine.

Il est à noter que tous les procédés connus à ce jour font intervenir sans exception une coupure de la catharanthine induite par une oxydation ou activation de cette dernière. Ces procédés sont mis en oeuvre dans des conditions contraignantes et ne permettent pas des rendements satisfaisants d'un produit suffisamment pur.

Le procédé selon la présente invention permet d'obtenir les vinca-alcaloïdes de formule générale (I) et en particulier l'anhydrovinblastine, en mettant en oeuvre des conditions opératoires peu contraignantes, avec d'excellents rendements et une grande pureté. Le procédé selon la présente invention permet l'obtention de l'anhydrovinblastine dans sa forme naturellement active, sans trace de l'épimére 16'-R de l'anhydrovinblastine.

La présente invention concerne donc un procédé de préparation d'un produit [A] répondant à la formule générale (I). 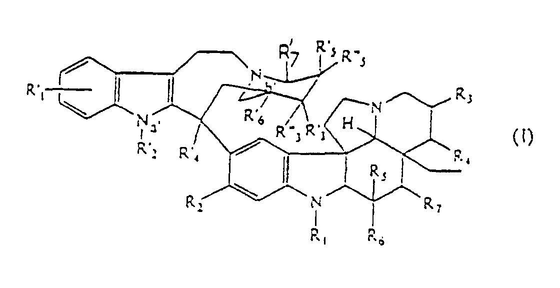 dans laquelle :
- R'₁ représente un atome d'hydrogène ou un radical alcoxy, acyle, formyle ou halogénoacyle,
- R'₂ représente un atome d'hydrogène ou un radical alcoyle,
- R'₃ et R"₃ sont identiques ou différents l'un de l'autre et représentent chacun indépendamment un atome d'hydrogène ou un radical hydroxyle, alcanoyloxyle, ou bien R'₃ et R"₃ forment ensemble un groupement carbonyle, ou bien R'₃ et R'₅ forment ensemble un pont époxy ou une double liaison,
- R'₄ représente un atome d'hydrogène ou un radical alcoyloxycarbonyle, hydroxyméthyle ou alcanoyloxyméthyle, de préférence alcoyloxycarbonyle,
- R'₅ et R"₅ sont identiques ou différents l'un de l'autre et représentent chacun indépendamment un atome d'hydrogène ou un radical hydroxyle, alcanoyloxyle, éthyle ou hydroxy-2-éthyle,
- R'₆ représente un atome d'hydrogène ou un radical éthyle, hydroxy-2-éthyle ou acétyle,
- R'₇ représente un atome d'hydrogène ou un radical cyanure,
- R₁ représente un atome d'hydrogène ou un radical alcoyle, formyle ou acyle, de préférence hydrogène ou alcoyle,
- R₂ représente un atome d'hydrogène ou un radical alcoxy,
- R₃ représente un atome d'hydrogène ou un radical hydroxyle, alcanoyloxyle, ou bien R₃ et R₄ forment ensemble un pont époxy ou une double liaison,
- R₄ représente un atome d'hydrogène ou un radical hydroxyle, alcanoyloxyle, ou bien R₄ et R₅ forment ensemble un pont époxy,
- R₆ représente un radical alcoyioxycarbonyle, hydrazido, acétamido, hydroxyméthyle ou alcanoyloxyméthyle,
- R₅ et R₇ représentent un atome d'hydrogène ou un radical hydroxyle ou alcanoyloxyle, ainsi que leurs sels d'addition avec les acides et leurs sels d'ammonium quaternaire, par réaction d'un produit [c] répondant à la formule générale (II) : 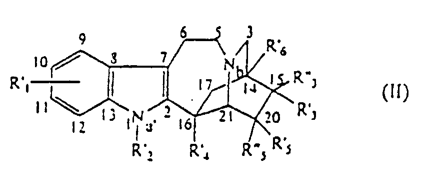
dans laquelle R'₁, R'₂, R'₃, R"₃, R'₄, R'₅, R"₅ et R'₆ sont définis comme précédemment,
avec un produit [v] répondant à la formule générale (III): 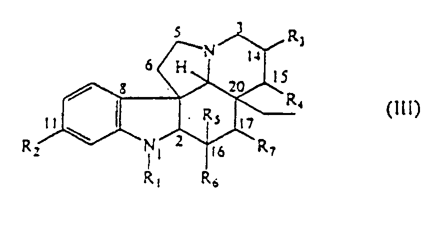 dans laquelle R₁, R₂, R₃, R₄, R₅, R₆ et R₇ sont définis comme précédemment,
ce procédé étant caractérisé par le fait que l'on choisit les conditions réactionnelles telles que le produit [v] soit oxydé afin d'obtenir un intermédiaire [i] de formule générale (IV) : 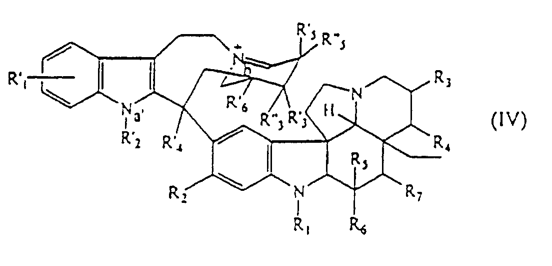 dans laquelle R₁, R₂, R₃, R₄, R₅, R₆, R₇ et R'₁, R'₂, R'₃, R"₃, R'₄, R'₅, R"₆ et R'₆ sont définis comme précédemment, et en ce que l'on soumet le produit (i) de formule (IV) soit à une réduction, soit à une cyanation afin d'obtenir le produit [A] de formule (I).

L'oxydation du produit (v) peut être effectuée par tous moyens connus. On peut citer comme exemple les voies photochimiques, organo-métalliques et électrochimiques. De préférence, l'oxydation du produit (v) est effectuée par voie photochimique en présence de lumière et éventuellement d'un sensibilisateur organique ou inorganique.

Le sensibilisateur inorganique peut être un métal de transition ou un semi-conducteur, étant de préférence un complexe de ruthénium ou de l'oxyde de titane.

Le sensibilisateur organique est un colorant choisi de préférence dans le groupe comprenant les xanthates, les pyriliums, les pyridiniums, les flavines, les aromatiques, les cétones et les quinones ainsi que leurs sels, et plus préférentiellement encore choisi parmi la fluorescéine, le triphénylpyrilium, le 4-(4-méthoxyphényl)-2,6-diphénylpyrilium, le 2,6-bis(4-méthoxyphényl)-4-phénylpyrilium et le 2,4,6-tris(4-méthoxyphényl)pyrilium.

L'étape d'oxydation est effectuée en milieu acide, de préférence à un pH compris entre 0 et 7, plus préférentiellement encore entre 0,5 et 3.

L'étape de réduction du produit [i] peut être effectuée par tous moyens connus. De préférence, on utilise un borohydrure alcalin, plus préférentiellement encore le borohydrure de sodium.

L'étape de cyanation du produit [i] peut être effectuée par tous moyens connus. De préférence, cette étape est effectuée en milieu organique en présence d'une source d'ions cyanure non ou peu alcaline, et plus particulièrement en présence de cyanure de triméthylsilyle.

La Société Demanderesse a en effet découvert que le fait de choisir les conditions réactionnelles de façon à oxyder le produit (v) (et non pas le produit (c) comme décrit dans l'art antérieur), conduit à une réaction stéréo spécifique en ce qui concerne l'atome de carbone C_{16'} de l'intermédiaire [i], évitant ainsi une contamination par l'épimère non actif 16'-R. De plus, cette stéréospécificité est indépendante de la température du milieu réactionnel, simplifiant ainsi le procédé de préparation.

Lorsque l'oxydation de (v) est effectuée par voie photochimique, la lumière est comprise dans le spectre UV-visible, de préférence supérieure à 254 nm et plus préférentiellement supérieure à 400 nm.

L'invention concerne également un procédé pour la préparation de l'anhydrovinblastine par réaction de la catharanthine avec la vindoline, ce procédé étant caractérisé par le fait que l'on choisit les conditions réactionnelles telles que la vindoline soit oxydée afin d'obtenir un intermédiaire [i'] de formule (V) : 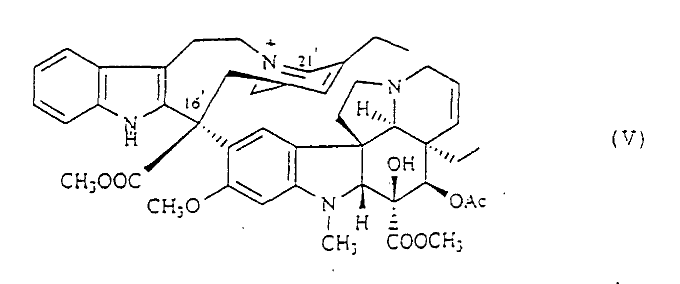 et en ce que l'on soumet l'intermédiaire [i'] de formule (V) à une réduction afin d'obtenir l'anhydrovinblastine.

L'invention concerne également des produits nouveaux [B] répondant à la formule générale (VI) : 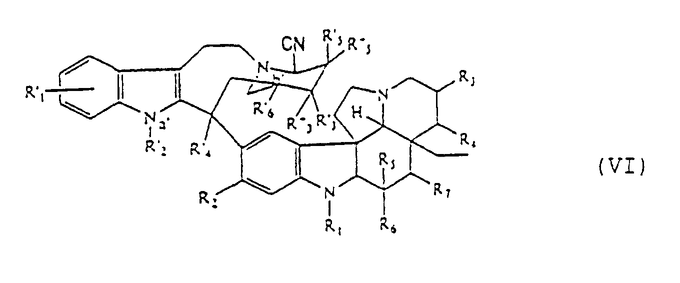 dans laquelle :
- R'₁ représente un atome d'hydrogène ou un radical alcoxy, acyle, formyle ou halogénoacyle,
- R'₂ représente un atome d'hydrogène ou un radical alcoyle,
- R'₃ et R"₃ sont identiques ou différents l'un de l'autre et représentent chacun indépendamment un atome d'hydrogène ou un radical hydroxyle, alcanorloxyle, ou bien R'₃ et R"₃ forment ensemble un groupement carbonyle, ou bien R'₃ et R'₅ forment ensemble une double liaison,
- R'₄, représente un atome d'hydrogène ou un radical alcoyloxycarbonyle, hydroxyméthyle ou alcanoyloxyméthyle,
- R'₅ et R"₅ sont identiques ou différents l'un de l'autre et représentent chacun indépendamment un atome d'hydrogène ou un radical hydroxyle, alcanovloxyle, éthyle ou hydroxy-2-éthyle,
- R'₆ représente un atome d'hydrogène ou un radical éthyle, hydroxy-2-éthyle ou acétyle,
- R₁ représente un atome d'hydrogène ou un radical alcoyle, formyle ou acyle,
- R₂ représente un atome d'hydrogène ou un radical alcoxy,
- R₃ représente un atome d'hydrogène ou un radical hydroxyle, alcanoyloxyle, ou bien R₃ et R₄ forment ensemble un pont époxy ou une double liaison,
- R₄ représente un atome d'hydrogène ou un radical hydroxyle, alcanoyloxyle, ou bien R₄ et R₅ forment ensemble un pont époxy,
- R₆ représente un radical alcoyloxycarbonyle, hydrazido, acétamido, hydroxyméthyle ou alcanoyloxyméthyle,
- R₅ et R₇ représentent un atome d'hydrogène ou un radical hydroxyle ou alcanoyloxyle.

Il s'agit de nouveaux dérivés de vinca-alcaloïdes dans lesquels le carbone à la position 21' porte un radical cyanure. Ces produits ont l'avantage d'être facilement fonctionnalisés, permettant ainsi l'obtention de molécules nouvelles potentiellement intéressantes sur le plan cytotoxique.

Ces produits [B] peuvent être fonctionnalisés aussi bien par des composés nucléophiles que par des composés électrophiles.

L'invention concerne en outre un produit nouveau [D] de formule VII :

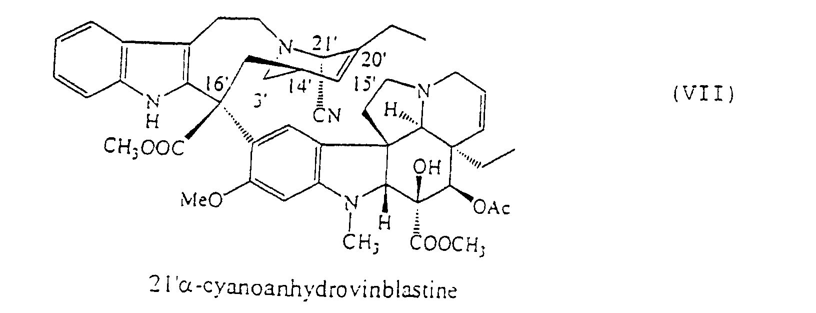

Il s'agit d'un dérivé de l'anhydrovinblastine, à savoir la 21'α-cyanoanhydrovinblastine. En effet la Société Demanderesse a mis au point un procédé de fabrication de la 21'α-cyanoanhydrovinblastine, une molécule qui peut être facilement fonctionnalisée permettant ainsi l'obtention des dérivés nouveaux et potentiellement intéressants sur le plan cytotoxique.

La 21'α-cyanoanhydrovinblastine peut être fonctionnalisée aussi bien par des composés nucléophiles que par des composés électrophiles. Ainsi, la gamme des dérivés potentiellement accessibles est très large.

Ce nouveau composé [Dj est également très intéressant car il est relativement stable, ce qui permet de l'isoler et de l'obtenir pur sous forme cristalline. L'avantage d'obtenir des cristaux d'un précurseur des dérivés est de permettre des réactions de fonctionnalisation de ce précurseur dans un milieu réactionnel propre, exempt de toutes traces de résidu de réactifs.

La 21'α-cyanoanhydrovinblastine peut permettre l'obtention des dérivés, mono ou poly substitués sur les positions C_{15'}, C₂₀ et C_{21'} de l'anhydrovinblastine ou de ces dérivés.

L'invention concerne également un procédé pour la préparation de la 21'α-cyanoanhydrovinblastine par réaction de la catharanthine avec la vindoline, ce procédé étant caractérisé par le fait que l'on choisit les conditions réactionnelles telles que la vindoline soit oxydée afin d'obtenir un intermédiaire [i'] de formule (V) et en ce que l'on soumet l'intermédiaire [i'] à une cyanation afin d'obtenir le produit [D].

La cyanation est effectuée de préférence en milieu organique en présence d'une source d'ions cyanures non ou peu alcaline, comme par exemple du cyanure de triméthylsilyle.

L'invention sera mieux comprise à l'aide des exemples non-limitatifs qui suivent.

### Exemple 1 :

Dans 25 ml d'acide chlorhydrique 0,1N sont additionnés 0,473 mmol de chlorhydrate de catharanthine (176 mg), 0,473 mmol de vindoline (216 mg) et 0,473 mmol d'hydrogénosulfate de triphénylpyrilium (192 mg). L'ensemble est irradié par de la lumière de longueur d'onde λ > 400 nm dans une fiole d'irradiation en pyrex, sous atmosphère d'oxygène. Après 6h30 d'irradiation, le milieu est extrait par du dichlorométhane.

La phase aqueuse est alors réduite à 0°C par un excès de borohydrure de sodium (2 éq.) en solution dans 3 ml de carbonate de sodium molaire ajouté goutte à goutte. Le mélange réactionnel est ensuite extrait par du dichlorométhane, séché et évaporé sous pression réduite à 20°C. Le résidu est solubilisé dans de l'acide chlorhydrique 0,5N et lavé par de l'éther diéthylique. La phase aqueuse est alcalinisée par de l'ammoniaque et extraite par du dichlorométhane.

L'unique produit du résidu (337 mg, 0,426 mmol, 90%) est recristallisé dans l'éthanol absolu. On récupère 290 mg de cristaux blancs d'anhydrovinblastine (0,366 mmol ; rendement: 77%).

### Exemple 2 :

Dans 50 ml d'acide sulfurique 0,1N sont additionnés 0,537 mmol de chorhydrate de catharanthine (200 mg), 0,537 mmol de vindoline (245 mg), 0,054 mmol de diméthyl viologène (14 mg) et 0,028 mmol d'hydrogénosulfate de triphénylpyrilium (11mg). L'ensemble est irradié par de la lumière de longueur d'onde λ>400 nm dans une fiole d'irradiation en pyrex, sous atmosphère d'oxygène. La réaction est terminée au bout de 2h30 d'irradiation.

La phase aqueuse est alors réduite à 0°C par un excès de borohydrure de sodium (2 éq.) en solution dans 10 ml de carbonate de sodium molaire ajouté goutte à goutte. Le mélange réactionnel est ensuite extrait par du dichlorométhane, séché et évaporé sous pression réduite à 20°C. Le résidu est solubilisé dans de l'acide chlorhydrique 0,5N et lavé par de l'éther diéthylique. La phase aqueuse est alcalinisée par de l'ammoniaque et extraite par du dichlorométhane.

L'unique produit du résidu (403 mg, 0,509 mmol, 95%) est recristallisé dans l'éthanol absolu. On récupère 340 mg de cristaux blancs d'anhydrovinblastine (0,430 mmol; rendement : 80%).

### Exemple 3 :

Dans 50 ml d'acide sulfurique 0,1N sont additionnés 0,537 mmol de chlorhydrate de catharanthine (200 mg), 0,537 mmol de vindoline (245 mg) et 0,054 mmol d'hydrogénosulfate de 2,6-bis(4-méthoxyphényl)-4-phénylpyrilium (25 mg). L'ensemble est irradié par de la lumière de longueur d'onde λ>400 nm dans une fiole d'irradiation en pyrex, sous atmosphère d'oxygène. La réaction est terminée au bout de 2h d'irradiation.

La phase aqueuse est alors réduite à 0°C par un excès de borohydrure de sodium (2 éq.) en solution dans 10 ml de carbonate de sodium molaire ajouté goutte à goutte. Le mélange réactionnel 1 est ensuite extrait par du dichlorométhane, séché et évaporé sous pression réduite à 20°C. Le résidu est solubilisé dans de l'acide chlorhydrique 0,5N et lavé par de l'éther diéthylique. La phase aqueuse est alcanisée par de l'ammoniaque et extraite par du dichlorométhane.

L'unique produit du résidu (408 mg, 0,516 mmol, 96%) est recristallisé dans l'éthanol absolu. On récupère 340 mg de cristaux blancs d'anhydrovinblastine (0,430 mmol; rendement : 80%).

### Exemple 4 :

Dans 50 ml d'acide sulfurique 0,1N sont additionnés 0,806 mmol de chlorhydrate de catharanthine (300 mg), 0,806 mmol de vindoline (367 mg) et 0,040 mmol de fluorescéine (5%). L'ensemble est irradié par de la lumière de longueur d'onde λ>400 nm dans une fiole d'irradiation en pyrex, sous atmosphère d'oxygène. La réaction est terminée au bout de 3h d'irradiation.

La phase aqueuse est alors réduite à 0°C par un excès de borohydrure de sodium (2 éq.) en solution dans 3 ml de carbonate de sodium molaire ajouté goutte à goutte. Le mélange réactionnel est ensuite extrait par du dichlorométhane, séché et évaporé sous pression réduite à 20°C.

L'unique produit du résidu (613 mg, 0,774 mmol, 96%) est recristallisé dans l'éthanol absolu. On récupère 523 mg de cristaux blancs d'anhydrovinblastine (0,661 mmol; rendement : 82%).

### Exemple 5 :

Dans 50 ml d'acide chlorhydrique 0,1N sont additionnés 0,268 mmol de chlorhydrate de catharanthine (100 mg), 0,263 mmol de vindoline (122 mg) et 0,029 mmol (5%) de sel de sodium de riboflavine 5'-phosphate dihydrate (FMN). L'ensemble est irradié par de la lumière de longueur d'onde λ>400 nm dans une fiole d'irradiation en pyrex, sous atmosphère d'oxygène. La réaction est terminée au bout de 4 h d'irradiation.

La phase aqueuse est alors réduite à 0°C par un excès de borohydrure de sodium (2 éq.) en solution dans 3 ml de carbonate de sodium molaire ajouté goutte à goutte. Le mélange réactionnel est ensuite extrait par du dichlorométhane, séché et évaporé sous pression réduite à 20°C.

Après recristallisation dans l'éthanol absolu du produit du résidu (171 mg, 0,216 mmol, 80%), on récupère 140 mg de cristaux blancs d'anhydrovinblastine (0,176 mmol; rendement : 66%).

### Exemple 6 :

Dans 60 ml d'acide chlorhydrique 0,1N sont additionnés 0,577 mmol de chlorhydrate de catharanthine (215 mg), 0,577 mmol de vindoline (263 mg) et 0,029 mmol (5%) de bis chlorure de *N*,*N*'-diméthyl-2,7-diazapyrénium (DAP²⁺). L'ensemble est irradié par de la lumière de longueur d'onde λ>400 nm dans une fiole d'irradiation en pyrex, sous atmosphère d'oxygène. La réaction est terminée au bout de 3h30 d'irradiation.

La phase aqueuse est alors réduite à 0°C par un excès de borohydrure de sodium (2 éq.) en solution dans 3ml de carbonate de sodium molaire ajouté goutte à goutte. Le mélange réactionnel est ensuite extrait par du dichlorométhane, séché et évaporé sous pression réduite à 20°C.

Après purification par chromatographie éclair sur gel de silice du résidu (379 mg, 0,479 mmol, 83%) et recristallisation dans l'éthanol absolu, on récupère 274 mg de cristaux blancs d'anhydrovinblastine (0,346 mmol ; rendement : 60%).

### Exemple 7 :

Dans 25 ml d'acide chlorhydrique 0,1N sont additionnés 0,357 mmol de chlorhydrate de catharanthine (120 mg), 0,357 mmol de vindoline (152 mg) et 2 g de TiO₂. L'ensemble est irradié par de la lumière de longueur d'onde λ>345 nm dans une fiole d'irradiation en pyrex, sous atmosphère d'oxygène. Après 2h d'irradiation, le milieu est extrait par du dichlorométhane.

La phase aqueuse est alors réduite à 0°C par un excès de borohydrure de sodium (2 éq.) en solution dans 3 ml de carbonate de sodium molaire ajouté goutte à goutte. Le mélange réactionnel est ensuite excrait par du dichlorométhane, séché et évaporé sous pression réduite à 20°C.

Après purification par chromatographie éclair sur gel de silice, on obtient 85 mg d'anhydrovinblastine (0,107 mmol ; rendement : 30%).

### Exemple 8 :

Dans 60 ml d'acide sulfurique 0,1N sont additionnés 0,448 mmol de chlorhydrate de catharanthine (166 mg), 0,448 mmol de vindoline (204 mg) et une quantité catalytique (5%) de rhodamine 6G. L'ensemble est irradié par de la lumière de longueur d'onde λ>400 nm dans une fiole d'irradiation en pyrex, sous atmosphère d'oxygène. Après 3h30 d'irradiation, le milieu est extrait par du dichlorométhane.

La phase aqueuse est alors réduite à 0°C par un excès de borohydrure de sodium (2éq.) en solution dans 3 ml de carbonate de sodium molaire ajouté goutte à goutte. Le mélange réactionnel est ensuite extrait par du dichlorométhane, séché et évaporé sous pression réduite à 20°C.

Après purification par chromatographie éclair sur gel de silice, on obtient 124 mg d'anhdrovinblastine (0,157 mmol ; rendement : 35%).

### Exemple 9 :

Dans 50 ml d'acide chlorhydrique 0,1N sont additionnés 0,537 mmol de chlorhydrate de catharanthine (200 mg), 0,537 mmol de vindoline (245 mg) et 0,054 mmol de diméthyl viologène (14 mg). L'ensemble est irradié par de la lumière de longueur d'onde λ>360 nm dans une fiole d'irradiation en pyrex, sous atmosphère d'oxygène. La réaction est terminée au bout de 2h d'irradiation.

La phase aqueuse est alors réduite à 0° C par un excès de borohydrure de sodium (2 éq.) en solution dans 3 ml de carbonate de sodium molaire ajouté goutte à goutte. Le mélange réactionnel est ensuite extrait par du dichlorométhane, séché et évaporé sous pression réduite à 20°C.

L'unique produit du résidu (391 mg, 0,494 mmol, 92%) est recristallisé dans l'éthanol absolu. On récupère 345 mg de -cristaux blancs d'anhydrovinblastine (0,435 mmol; rendement : 81%).

### Exemple 10 :

Dans 50 ml d'acide chlorhydrique 0,1N sont additionnés 0,537 mmol de chlorhydrate de catharanthine (200 mg), 0,537 mmol de vindoline (245 mg). L'ensemble est irradié par de la lumière de longueur d'onde λ>360 nm dans une fiole d'irradiation en pyrex, sous atmosphère d'oxygène. La réaction est terminée au bout de 2 h d'irradiation.

La phase aqueuse est alors réduite à 0°C par un excès de borohydrure de sodium (2 éq.) en solution dans 3 ml de carbonate de sodium molaire ajouté goutte à goutte. Le mélange réactionnel est ensuite extrait par du dichlorométhane, séché et évaporé sous pression réduite à 20°C.

L'unique produit du résidu (383 mg, 0,483 mmol, 90%) est recristallisé dans l'éthanol absolu. On récupère 332 mg de cristaux blancs d'anhydrovinblastine (0,419 mmol; rendement:78%).

### Exemple 11 :

### Préparation de la 21'α-cyanoanhydrovinblastine.

Dans 50 ml d'acide sulfurique 0,1N sont additionnés 0,537 mmol de chlorhydrate de catharanthine (200 mg), 0,537 mmol de vindoline (245 mg), 0,054 mmol de diméthyl viologène (14 mg) et 0,028 mmol d'hydrogénosulfate de triphénylpyrilium (11 mg). L'ensemble est irradié par de la lumière de longueur d'onde λ>400 nm dans une fiole d'irradiation en pyrex, sous atmosphère d'oxygène. La réaction est terminée au bout de 2h30 d'irradiation.

La phase aqueuse est alors saturée avec du tétrafluorobrate de lithium puis extraite par du dichlorométhane. Une solution de 15 ml de dichlorométhane contenant 100µl (1.34 mmol, 2.5 éq.) de cyanure de triméthylsilyle, TMSCN, est alors additionnée au milieu réactionnel. La phase organique est lavée par une solution 0.1M de carbonate de sodium, séchée et évaporée sous pression réduite à 20°C.

L'unique produit du résidu (403 mg, 0,509 mmol, 95%) est recristallisé dans l'isopropanol absolu. On récupère 340 mg de cristaux blancs de 21'α-cyanoanhydrobinblastine (0,430 mmol ; rendement : 80%).

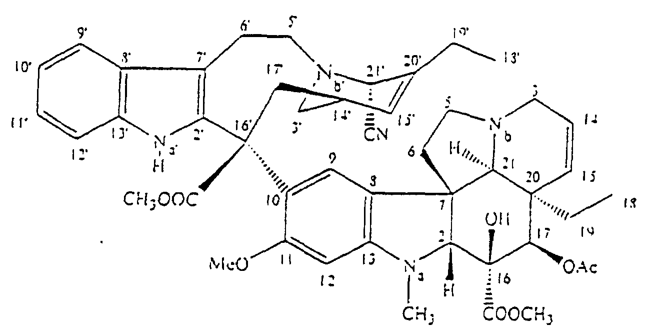

C₄₇H₅₅N₅O₈

PF 212°C(iPrOH)
IR film 3450, 2950, 2220, 1740, 1610 cm⁻¹ ;
SM M/z (intensité relative)818(MH+,3),122(100), 108(21);
RMN ¹H(500 MHz, CDCl3) 9.78(s,1H,OH), 8. 04 (s,1H,Na'H), 7,51(1H,H-9'), 7,16(1H,H-11'), 7.13(1H, H-12'), 7.12(1H,H-10'), 6.63(s,1H,H-9), 6.13(s,1H,H-12), 5.85(m,1H,H-14), 5.47(s,1H,H_{α}-17), 5.54(m,1H,H-15'), 5.30(m,1H,H-15), 4.18(1H,H_{β}-2), 3.60(s,3H,C16'-COOCH₃), 3.38(1H,H_{β}-3), 3.35(1H,H_{β}-3'), 3.31(1H,H_{β}-5), 3.25(1H,H_{β}-6'), 3.24(m,1H,H_{β}-5'), 3.15(1H, H_{β}-17'), 3.14(m,1H,H_{α}-5'), 3.12(1H,H_{α}-6'), 2.82(1H,H_{α}-3), 2.72(s,3H,NaCH₃), 2.66(s,1H,H_{α}-21), 2.62(1H,H_{α}-3'), 2.46(1H,H_{α}-5), 2.40(1H,H_{α}-17'), 2.20(1H,H_{β}-6), 2.11(s,3H,CH₃-COO), 2.11(1H,H-19'), 2.03(1H,H-19'), 1.80(1H,H_{α}-6), 1.80(1H,H-19), 1.35(1H,H-19), 1.21(m,1H,H-14'), 1.04(3H,H-18'), 0.81(3H,H-18);
RMN ¹³C (125MHz,CDCl3) 174.69(C_{16'}-COOCH₃), 171.74 (C₁₆-COOCH₃), 171.03130.01(C₁₅), 129.34(C_{8'}), 129.16(C_{15'}), 124.63(C₁₄), 123.48(C₉), 123.24(C₈), 122.49(C_{11'}), 121.00(C₁₀), 119.21(C_{10'}), 119.21(CN), 118.35(C_{9'}, 115.65(C_{7'}), 110.64(C₁₁-OCH₃), 55.40(C_{16'}), 53.30(C₇); 52.46(C_{16'}-COOCH₃), 52.30(C₁₆-COOCH₃), 52.26(C_{5'}), 50.50(C5), 50,41(C₃), 44.86(C₆), 44.48(C_{3'}), 42.76(C₂₀), 38.32(Na-CH₃), 34.00(C_{17'}), 33.28(C_{14'}), 30.92(C₁₉), 28.63(C_{6'}), 25.92(C_{19'}), 21.19(CH₃-COO), 11.86(C_{18'}), 8.50(C₁₈).

## Revendications

1. Procédé de préparation d'un produit [A] répondant à la formule générale (I) 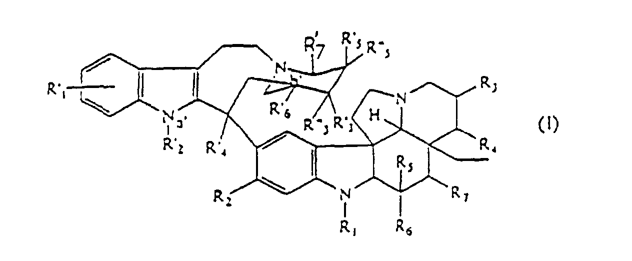 dans laquelle :
- R'₁ représente un atome d'hydrogène ou un radical alcoxy, acyle, formyle ou halogénoacyle,
- R'₂ représente un atome d'hydrogène ou un radical alcoyle,
- R'₃ et R"₃ sont identiques ou différents l'un de l'autre et représentent chacun indépendamment un atome d'hydrogène ou un radical hydroxyle, alcanoyloxyle, ou bien R'₃ et R"₃ forment ensemble un groupement carbonyle, ou bien R'₃ et R'₅ forment ensemble un pont époxy ou une double liaison,
- R'₄ représente un atome d'hydrogène ou un radical alcoyloxycarbonyle, hydroxyméthyle ou alcanoyloxyméthyle, de préférence alcoyloxycarbonyle,
- R'₅ et R"₅ sont identiques ou différents l'un de l'autre et représentent chacun indépendamment un atome d'hydrogène ou un radical hydroxyle, alcanoyloxyle, éthyle ou hydroxy-2-éthyle,
- R'₆ représente un atome d'hydrogène ou un radical éthyle, hydroxy-2-éthyle ou acétyle,
- R'₇ représente un atome d'hydrogène ou un radical cyanure,
- R₁ représente un atome d'hydrogène ou un radical alcoyle, formyle ou acyle, de préférence hydrogène ou alcoyle,
- R₂ représente un atome d'hydrogène ou un radical alcoxy,
- R₃ représente un atome d'hydrogène ou un radical hydroxyle, alcanoyloxyle, ou bien R₃ et R₄ forment ensemble un pont époxy ou une double liaison,
- R₄ représente un atome d'hydrogène ou un radical hydroxyle, alcanoyloxyle, ou bien R₄ et R₅ forment ensemble un pont époxy,
- R₆ représente un radical alcoyloxycarbonyle, hydrazido, acétamido, hydroxyméthyle ou alcanoyloxyméthyle,
- R₅ et R₇ représentent un atome d'hydrogène ou un radical hydroxyle ou alcanoyloxyle,
ainsi que leurs sels d'addition avec les acides et leurs sels d'ammonium quaternaire, par réaction d'un produit [c] répondant à la formule générale (II) : 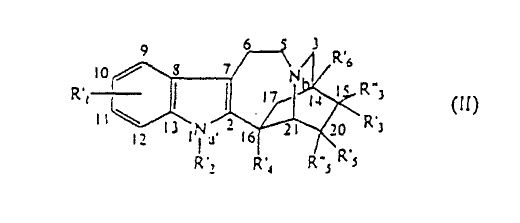 dans laquelle R'₁, R'₂, R'₃, R"₃, R'₄, R'₅, R"₅ et R'₆ sont définis comme précédemment,
avec un produit [v] répondant à la formule générale (III): 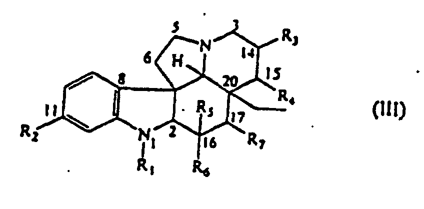 dans laquelle R₁, R₂, R₃, R'₄, R₅, R₆ et R₇ sont définis comme précédemment.
ce procédé étant **caractérisé par le fait que** l'on oxyde le produit [v] par tous moyens connus, notamment par voies photochimiques, organométalliques et électrochimique afin d'obtenir un intermédiaire [i] de formule générale (IV) : 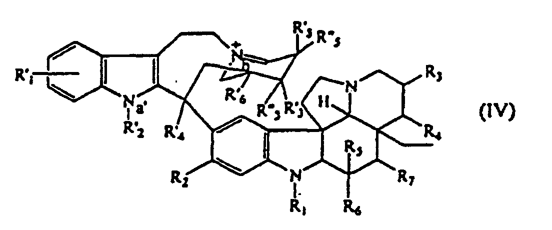 dans laquelle R₁, R₂, R₃, R₄, R₅, R₆, R₇ et R'₁, R'₂, R'₃, R"₃, R'₄, R'₅, R"₅ et R'₆ sont définis comme précédemment, et en ce que l'on soumet le produit (i) de formule (IV) soit à une réduction soit à une cyanation afin d'obtenir le produit [A].

2. Procédé selon la revendication 1, **caractérisé par le fait que** le produit (v) est oxydé par voie photochimique.

3. Procédé selon la revendication 1, **caractérisé par le fait que** le produit (v) est oxydé par voie organométallique.

4. Procédé selon la revendication 1, **caractérisé par le fait que** le produit (v) est oxydé par voie électrochimique.

5. Procédé selon l'une ou l'autre des revendications 1 et 2, **caractérisé par le fait que** l'oxydation par voie photochimique est effectuée en présence de lumière et éventuellement d'un sensibilisateur organique ou inorganique.

6. Procédé selon la revendication 5, **caractérisé par** la fait que le sensibilisateur inorganique est un métal de transition ou un semi-conducteur, de préférence un complexe de Ruthénium ou de l'oxyde de titane.

7. Procédé selon la revendication 5, **caractérisé par le fait que** le sensibilisateur organique est un colorant choisi de préférence dans le groupe comprenant les xanthates, les pyriliums, les pyridiniums, les flavines, les aromatiques, les cétones et les quinones, ainsi que leurs sels, et plus préférentiellement encore choisi parmi la fluorescéine, le triphénylpyrilium, le 4-(4-méthoxyphényl)-2,6-diphénylpyrilium, le 2,6-bis(4-méthoxyphényl)-4-phénylpyrilium et le 2,4,6-tris(4-méthoxyphényl)pyrilium.

8. Procédé selon la revendication 5, **caractérisé par le fait que** la lumière est d'une longueur d'onde comprise dans le spectre UV-visible, de préférence supérieure à 254 nm et plus préférentiellement supérieure à 400 nm.

9. Procédé selon l'une quelconque des revendications 1 à 8, **caractérisé par le fait que** l'étape de réduction est réalisée à l'aide d'un borohydrure alcalin, de préférence le borohydrure de sodium.

10. Procédé selon l'une quelconque des revendications 1 à 9, **caractérisé par le fait que** le produit [A] est l'anhydrovinblastine,
[c] est la catharanthine,
[v] est la vindoline,
et le produit [i] répond à la formule (V) :

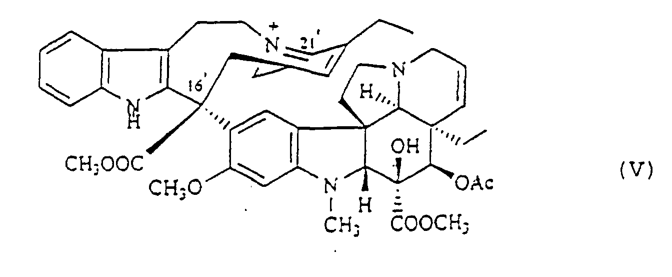

11. Produit nouveau [B], de formule générale (VI): 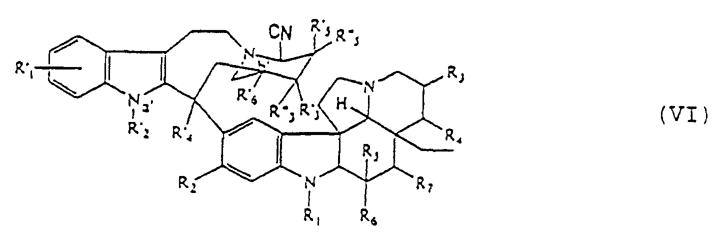 dans laquelle :
- R'₁ représente un atome d'hydrogène ou un radical alcoxy, acyle, formyle ou halogénoacyle,
- R'₂ représente un atome d'hydrogène ou un radical alcoyle,
- R'₃ et R"₃ sont identiques ou différents l'un de l'autre et représentent chacun indépendamment un atome d'hydrogène ou un radical hydroxyle, alcanoyloxyle, ou bien R'₃ et R"₃ forment ensemble un groupement carbonyle, ou bien R'₃ et R'₅ forment ensemble une double liaison,
- R'₄ représente un atome d'hydrogène ou un radical alcoyloxycarbonyle, hydroxyméthyle ou alcanoyloxyméthyle, de préférence alcoyloxycarbonyle,
- R'₅ et R"₅ sont identiques ou différents l'un de l'autre et représentent chacun indépendamment un atome d'hydrogène ou un radical hydroxyle, alcanoyloxyle, éthyle ou hydroxy-2-éthyle,
- R'₆ représente un atome d'hydrogène ou un radical éthyle, hydroxy-2-éthyle ou acétyle,
- R₁ représente un atome d'hydrogène ou un radical alcoyle, formyle ou acyle, de préférence hydrogène ou alcoyle,
- R₂ représence un atome d'hydrogène ou un radical alcoxy,
- R₃ représente un atome d'hydrogène ou un radical hydroxyle, alcanoyloxyle, ou bien R₃ et R₄ forment ensemble un pont époxy ou une double liaison,
- R₄ représente un atome d'hydrogène ou un radical hydroxyle, alcanoyloxyle, ou bien R₄ et R₅ forment ensemble un pont époxy,
- R₆ représente un radical alcoyloxycarbonyle, hydrazido, acétamido, hydroxyméthyle ou alcanoyloxyméthyle,
- R₅ et R₇ représentent un atome d'hydrogène ou un radical hydroxyle ou alcanoyloxyle.

12. Produit nouveau [D], de formule (VII)

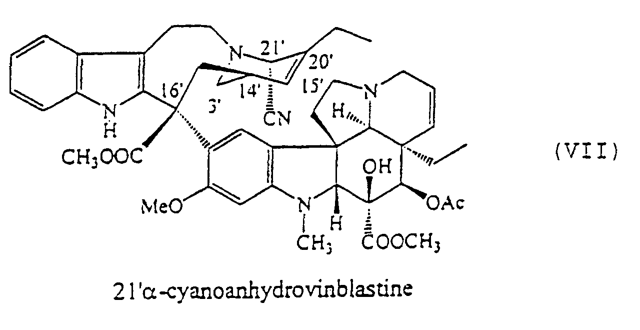

13. Procédé pour la préparation du produit nouveau [D] tel que défini à la revendication 12 par réaction de la catharanthine avec la vindoline, ce procédé étant **caractérisé par le fait que** l'on choisit les conditions réactionnelles telles que la vindoline soit oxydée afin d'obtenir un intermédiaire [i'] de formule (V) et en ce que l'on soumet l'intermédiaire [i'] à une cyanation afin d'obtenir le produit [D].

14. Procédé selon l'une quelconque des revendications 1 à 8 et 13, **caractérisé par le fait que** l'étape de cyanation est effectuée de préférence en milieu organique en présence d'une source d'ions cyanure non ou peu alcaline, et plus préférentiellement encore en présence de cyanure de triméthylsilyle.

## Patentansprüche

1. Verfahren zur Herstellung eines Produktes (A) entsprechend der allgemeinen Formel (I) 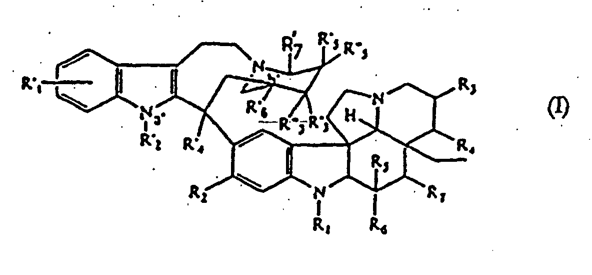 bei welcher.
- R'₁ ein Wasserstoffatom oder einen Alkoxy-, Acyl-, Formyl- oder Halogenacylrest darstellt,
- R'₂ ein Wasserstoffatom oder einen Alkylrest darstellt,
- R'₃ und R"₃ identisch oder voneinander verschieden sind und jedes unabhängig ein Wasserstoffatom oder einen Hydroxyl-, Alkanoyloxyl-Rest darstellt oder auch R'₃ oder R"₃ zusammen eine Carbonylgruppe bilden oder auch R'₃ und R'₅ gemeinsam eine Epoxybrücke oder eine Doppelbindung bilden,
- R'₄ ein Wasserstoffatom oder einen Alkyloxycarbonyl-Rest, ein Hydroxymethyl - oder ein Alkanoyloxymethyl-Rest, vorzugsweise einen Alkyloxycarbonyl-Rest darstellt,
- R'₅ und R"₅ identisch oder voneinander verschieden sind und jedes unabhängig ein Wasserstoffatom oder einen Hydroxyl-, ein Alkanoyloxyl-, Ethyl- oder Hydroxy-2-Ethyl-Rest darstellt,
- R'₆ ein Wasserstoffatom oder ein Ethyl-, Hydroxy-2-Ethyl- oder Acetyl-Rest darstellt,
- R'₇ ein Wasserstoffatom oder einen Cyanidrest darstellt,
- R₁ ein Wasserstoffatom oder einen Alkyl-, Formyl-, Aryl-Rest, vorzugsweise Wasserstoff oder Alkyl-Rest, darstellt,
- R₂ ein Wasserstoffatom oder einen Alkoxy-Rest darstellt,
- R₃ ein Wasserstoffatom oder einen Hydroxyl-, Alkanoyloxyl-Rest darstellt oder auch R₃ und R₄ gemeinsam eine Epoxybrücke oder eine Doppelbindung bilden,
- R₄ ein Wasserstoffatom oder einen Hydroxyl-, Alkanoyloxyl-Rest darstellt oder auch R₄ und R₅ gemeinsam eine Epoxybrücke bilden,
- R₆ einen Alkyloxycarbonyl-, Hydrazido-, Acetamido-, Hydroxymethyl- oder Alkanoyloxymethyl-Rest darstellt,
- R₅ und R₇ ein Wasserstoffatom oder einen Hydroxyl- oder Alkanoyloxyl-Rest darstellen,
ebenso wie ihre Additionssalze mit Säuren oder ihrer quaternären Ammoniumsalze, durch Reaktion von einem Produkt (c), entsprechend der allgemeinen Formel (II): 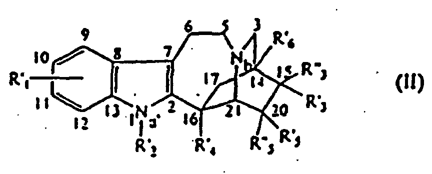 wobei R'₁, R'₂, R'₃, R"₃, R'₄, R'₅, R"₅ und R'₆ wie vorstehend definiert sind,
mit einem Produkt (v), entsprechend der allgemeinen Formel (III): 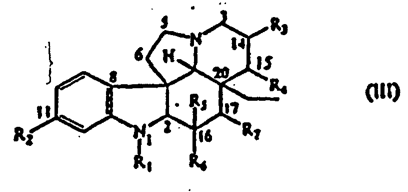 wobei R₁, R₂, R₃, R'₄, R₅, R₆ und R₇ wie vorstehend definiert sind;
wobei dieses Verfahren **dadurch gekennzeichnet ist, dass** man das Produkt (V) mit allen bekannten Mitteln, besonders auf photochemischen, organometallischen und elektrochemischen Wegen oxidiert, um ein Intermediat (i) der allgemeinen Formel (IV) zu erhalten: 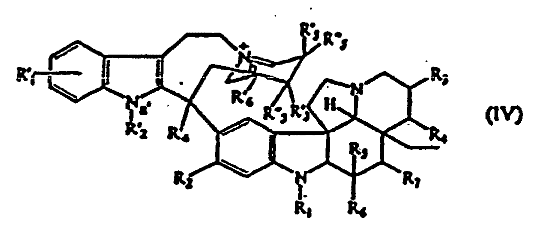 wobei R₁, R₂, R₃, R₄, R₅, R₆, R₇, und R'₁, R'₂, R'₃ R"₃, R'₄, R'₅, R"₅ und R'₆ wie oben definiert sind, und in welchem man das Produkt (i) der Formel (IV) entweder einer Reduktion oder einer Cyanierung unterzieht, um das Produkt (A) zu erhalten.

2. Verfahren nach Anspruch 1, **dadurch gekennzeichnet, dass** das Produkt (v) auf photochemischem Weg oxidiert wird.

3. Verfahren nach Anspruch 1, **dadurch gekennzeichnet, dass** das Produkt (v) auf organometallischem Weg oxidiert wird.

4. Verfahren nach Anspruch 1, **dadurch gekennzeichnet, dass** das Produkt (v) auf elektrochemischem Weg oxidiert wird.

5. Verfahren nach Anspruch 1 oder 2, **dadurch gekennzeichnet, dass** die Oxidation auf photochemischem Weg in Gegenwart von Licht und gegebenenfalls eines organischen oder anorganischen Sensibilisators bewirkt wird.

6. Verfahren nach Anspruch 5, **dadurch gekennzeichnet, dass** der anorganische Sensibilisator ein Übergangsmetall oder ein Halbleiter ist, vorzugsweise ein Ruthenium- oder Titanoxid-Komplex.

7. Verfahren nach Anspruch 5, **dadurch gekennzeichnet, dass** der organische Sensibilisator ein Farbstoff ist, vorzugsweise ausgewählt aus der Gruppe, umfassend die Xanthongenate, Pyridiniume, Pyriliume Flavine, Aromaten, Ketone und Chinone sowie ihre Salze, und noch stärker bevorzugt ausgewählt ist unter Fluoreszin, Triphenylpyrilium, 4-(4-Methoxyphenyl)-2,6-diphenylpyrilium, 2,6-Triphenylpyrilium, 4-(4-Methoxyphenyl)-2,6-diphenylpyrilium, 2,6-bis(4-Methoxyphenyl)-4-phenylpyrilium und 2,4,6-tris(4-Methoxyphenyl)pyrilium.

8. Verfahren nach Anspruch 5, **dadurch gekennzeichnet, dass** das Licht eine Wellenlänge innerhalb des im UV-sichtbaren Spektrums, vorzugsweise oberhalb von 254 nm und stärker bevorzugt oberhalb von 400 nm hat.

9. Verfahren nach einem der Ansprüche 1 bis 8, **dadurch gekennzeichnet, dass** der Reduktionsschritt mit Hilfe eines Alkaliborhydrids, vorzugsweise Natriumborhydrid, ausgeführt wird.

10. Verfahren nach einem der Ansprüche 1 bis 9, **dadurch gekennzeichnet, dass** das Produkt (A) Anhydrovinblastin ist,
(c) Catharanthin ist,
(v) Vindolin ist,
und das Produkt (i) der Formel (V) entspricht

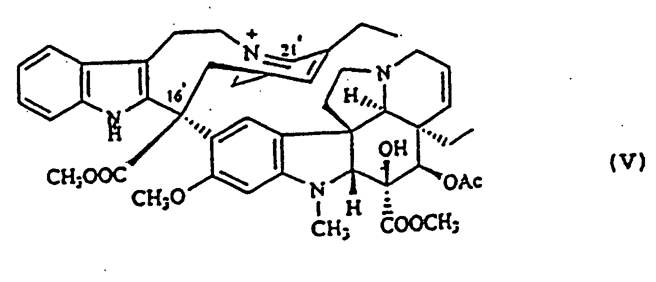

11. Neues Produkt (B) der allgemeinen Formel (VI): 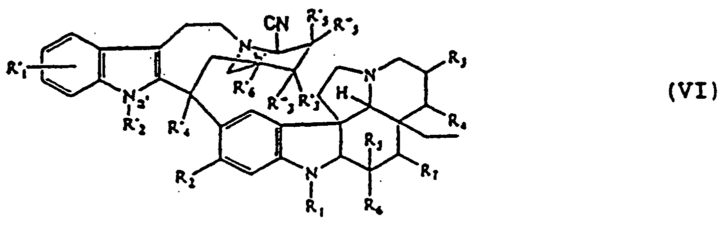 wobei:
- R₁, ein Wasserstoffatom oder ein Alkoxy-, Acyl-, Formyl- oder Halogenacyl-Rest darstellt,
- R'₂ ein Wasserstoffatom oder einen Alkylrest darstellt,
- R'₃ und R"₃ identisch oder voneinander verschieden sind und jedes unabhängig ein Wasserstoffatom oder ein Hydroxyl-, Alkanoyloxyl-Rest darstellt oder auch R'₃ und R"₃ gemeinsam eine Cabonylgruppe bilden oder auch R'₃ und R'₅ gemeinsam eine Doppelbindung bilden,
- R'₄ ein Wasserstoffatom oder einen Alkyloxycarbonyl-, Hydroxymethyl- oder Alcanoyloxymethyl-Rest, vorzugsweise Alkyloxycarbonyl-Rest, darstellt,
- R'₅ und R"₅ identisch oder voneinander verschieden sind und jedes unabhängig ein Wasserstoffatom oder einen Hydroxyl-, Alkanoyloxyl-, Ethyl- oder Hydroxy-2-Ethyl-Rest darstellt,
- R'₆ ein Wasserstoffatom oder einen Ethyl-, Hydroxy-2-Ethyl- oder Acetyl-Rest darstellt,
- R₁ ein Wasserstoffatom oder einen Alkyl-, Formyl- oder Acyl-Rest darstellt, vorzugsweise ein Wasserstoff oder Alkyl-Rest,
- R₂ ein Wasserstoffatom oder einen Alkoxy-Rest darstellt,
- R₃ ein Wasserstoffatom oder einen Hydroxyl-, Alkanoylozcyl-Rest darstellt, oder auch R₃ und R₄ gemeinsam eine Epoxybrücke oder eine Doppelbindung bilden,
- R₄ ein Wasserstoffatom oder einen Hydroxyl-, Alkanoyloxyl-Rest darstellt oder auch R₄ oder R₅ gemeinsam eine Epoxybrücke bilden,
- R₆ einen Allryloxycarbonyl-, Hydrazido-, Acetamido-, Hydroxymethyl- oder Alkanoyloxymethyl-Rest darstellt,
- R₅ und R₇ ein Wasserstoffatom oder einen Hydroxyl- oder Alkanoyloxyl-Rest darstellen.

12. Neues Produkt (D) der Formel (VII)

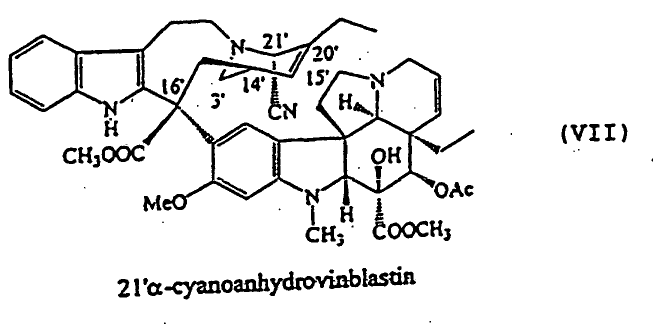

13. Verfahren zur Herstellung eines neuen Produkts (D), wie nach Anspruch 12 definiert, durch Reaktion von Catharanthin mit Vindolin, wobei dieses Verfahren **dadurch gekennzeichnet ist, dass** man die Reaktionsbedingungen so auswählt, dass Vindolin oxidiert wird, um ein Intermediat (i') der Formel (V) zu erhalten, und bei welchem man das Intermediat (i') einer Cyanierung unterzieht, um das Produkt (D) zu erhalten.

14. Verfahren nach einem der Ansprüche 1 bis 8 und 13, **dadurch gekennzeichnet, dass** der Cyanierungsschritt vorzugsweise in organischem Milieu in Gegenwart einer nicht oder wenig alkalischen Cyanidionen-Quelle durchgeführt wird, und noch stärker bevorzugt in Gegenwart von Trimethylsilylcyanid.

## Claims

1. A process for preparing a product (A) corresponding to the general formula (I), 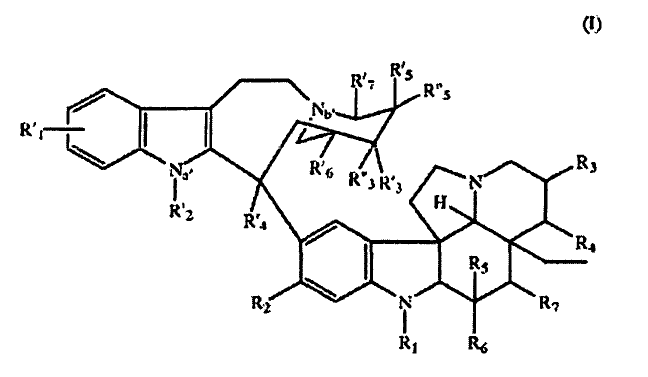 wherein:
- R'₁ represents a hydrogen atom or an alkoxy, acyl, formyl or halogenoacyl group,
- R'₂ represents a hydrogen atom or an alkoyl group,
- R'₃ and R"₃ are identical or different and each independently represents a hydrogen atom or a hydroxyl or alkanoyloxyl group, or else R'₃ and R"₃ together form a carbonyl group, or else R'₃ and R'₅ together form an epoxy bridge or a double bond,
- R'₄ represents a hydrogen atom or an alkoyloxycarbonyl, hydroxymethyl or alkanoyloxymethyl group, preferably an alkoyloxycarbonyl group,
- R'₅ and R"₅ are identical or different and each independently represents a hydrogen atom or a hydroxyl, alkanoyloxyl, ethyl or 2-hydroxyethyl group,
- R'₆ represents a hydrogen atom or an ethyl, 2-hydroxyethyl or acetyl group,
- R'₇ represents a hydrogen atom or a cyanide group,
- R₁ represents a hydrogen atom or an alkoyl, formyl or acyl group, preferably hydrogen or an alkoyl group,
- R₂ represents a hydrogen atom or an alkoxy group,
- R₃ represents a hydrogen atom or a hydroxyl or alkanoyloxyl group, or else R₃ and R₄ together form an epoxy bridge or a double bond,
- R₄ represents a hydrogen atom or a hydroxyl or alkanoyloxyl group, or else R₄ and R₅ together form an epoxy bridge,
- R₆ represents an alkoyloxycarbonyl, hydrazido, acetamido, hydroxymethyl or alkanoyloxymethyl group,
- R₅ and R₇ represent a hydrogen atom or a hydroxyl or alkanoyloxyl group,
as well as their addition salts with acids and their quaternary ammonium salts, by reacting a product (c) corresponding to the general formula (II): 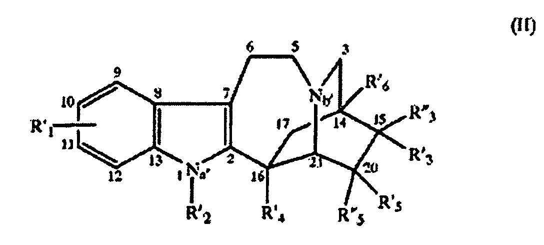 wherein R'₁, R'₂, R'₃, R''₃, R'₄ R'₅, R''₅ and R'₆ are defined as above,
with a product (v) corresponding to the general formula (III) : 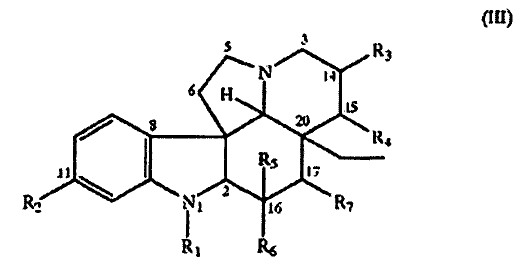 wherein R₁, R₂, R₃, R'₄, R₅, R₆ and R₇, are defined as above, said process being **characterized in that** the product (v) is oxidised by all known means, especially photochemically, organometallically, and electrochemically, in order to obtain an intermediate (i) of the general formula (IV): 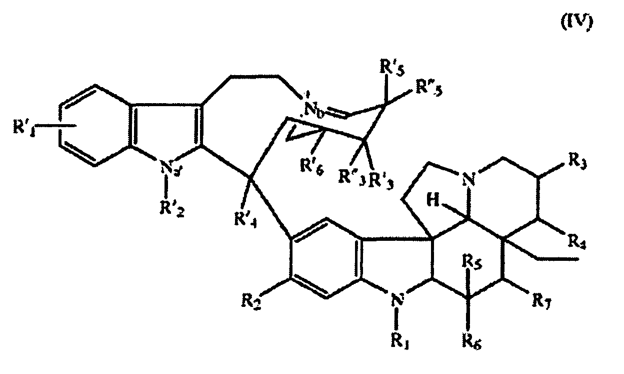 wherein R₁, R₂, R₃, R₄, R₅, R₆, R₇, and R'₁, R'₂, R'₃, R"₃, R'₄, R'₅, R"₅ and R'₆ are defined as above, and **in that** the product (i) of formula (IV) is subjected either to reduction or to cyanation in order to obtain product (A).

2. A process according to Claim 1, **characterised in that** the product (v) is oxidised photochemically.

3. A process according to Claim 1, **characterised in that** the product (v) is oxidised organometallically.

4. A process according to Claim 1, **characterized in that** the product (v) is oxidised electrochemically.

5. A process according to one or other of Claims 1 and 2, **characterised in that** photochemical oxidation is performed in the presence of light and optionally of an organic or inorganic sensitizer.

6. A process according to Claim 5, **characterized in that** the inorganic sensitizer is a transition metal or a semiconductor, preferably a ruthenium complex or titanium oxide.

7. A process according to Claim 5, **characterised in that** the organic sensitizer is a colorant, preferably selected from the group consisting of xanthates, pyriliums, pyridiniums, flavines, aromatic compounds, ketones and quinones, as well as their salts, and more preferably chosen from among fluorescein, triphenyl-pyrilium, 4-(4-methoxyphenyl)-2,6-diphenylpyrilium, 2,6-bis-(4-methoxyphenyl)-4-phenylpyrilium and 2,4,6-tris-(4-methoxyphenyl)-pyrilium.

8. A process according to Claim 5, **characterised in that** the light has a wavelength in the UV/visible spectrum, preferably greater than 254 nm and more preferably greater than 400 nm.

9. A process according to any one of Claims 1 to 8, **characterised in that** the reduction step is performed with an alkaline borohydride, preferably sodium borohydride.

10. A process according to any one of Claims 1 to 9, **characterized in that** the product (A) is anhydrovinblastine,
(c) is catharanthine,
(v) is vindoline,
and the product (i) corresponds to the formula (V):

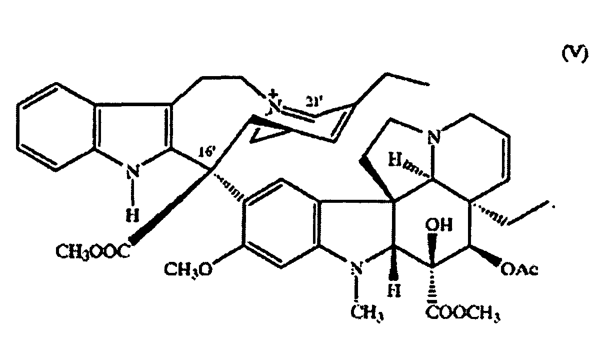

11. A new product (B), of the general formula (VI) : 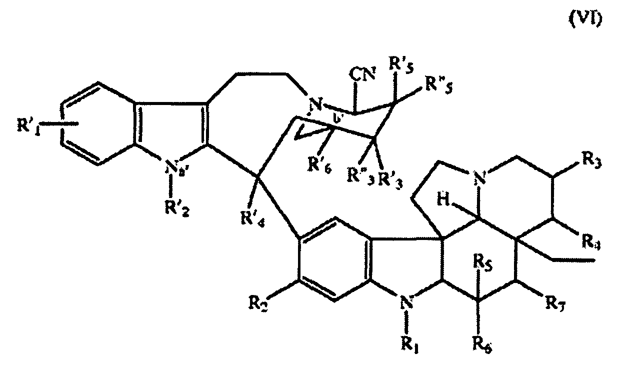 wherein:
- R'₁ represents a hydrogen atom or an alkoxy, acyl, formyl or halogenoacyl group,
- R'₂ represents a hydrogen atom or an alkoyl group,
- R'₃ and R''₃ are identical or different and each independently represents a hydrogen atom or a hydroxyl or alkanoyloxyl group, or else R'₃ and R"₃ together form a carbonyl group, or else R'₃ and R'₅ together form a double bond,
- R'₄ represents a hydrogen atom or an alkoyloxycarbonyl, hydroxymethyl or alkanoyloxymethyl group, preferably an alkoyloxycarbonyl group,
- R'₅ and R"₅ are identical or different and each independently represents a hydrogen atom or a hydroxyl, alkanoyloxyl, ethyl or 2-hydroxyethyl group,
- R'₆ represents a hydrogen atom or an ethyl, 2-hydroxyethyl or acetyl group,
- R₁ represents a hydrogen atom or an alkoyl, formyl or acyl group, preferably hydrogen or an alkoyl group,
- R₂ represents a hydrogen atom or an alkoxy group,
- R₃ represents a hydrogen atom or a hydroxyl or alkanoyloxyl group, or else R₃ and R₄ together form an epoxy bridge or a double bond,
- R₄ represents a hydrogen atom or a hydroxyl or alkanoyloxyl group, or else R₄ and R₅ together form an epoxy bridge,
- R₆ represents an alkoyloxycarbonyl, hydrazido, acetamido, hydroxymethyl or alkanoyloxymethyl group,
- R₅ and R₇ represent a hydrogen atom or a hydroxyl or alkanoyloxyl group.

12. A new product (D) of the formula (VII)

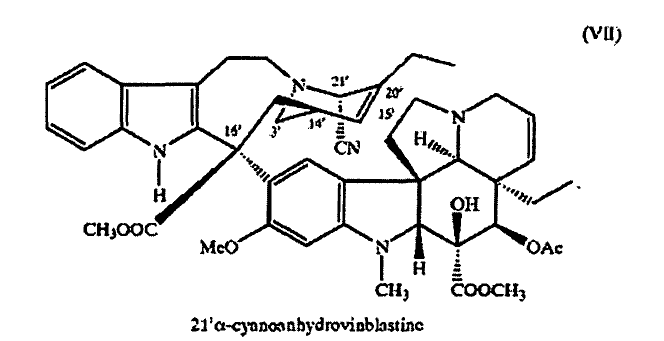

13. A process for preparing the new product (D) as defined in Claim 12, by reacting catharanthine with vindoline, said process being **characterised in that** the reaction conditions are chosen so that vindoline is oxidised in order to obtain intermediate (i') of formula (V) and **in that** the intermediate (i') is subjected to cyanation in order to obtain the product CD).

14. A process according to any one of Claims 1 to 8 and 13, **characterised in that** the cyanation step is preferably performed in an organic medium in the presence of a source of cyanide ions which is not alkaline or is slightly alkaline and more preferably in the presence of trimethylsilyl cyanide.
